# EUROPÄISCHE PATENTSCHRIFT

(11) **EP 2 593 164 B1**
(45) Veröffentlichungstag und Bekanntmachung des Hinweises auf die Patenterteilung: **22.03.2017**
(21) Anmeldenummer: 11730632.4
(22) Anmeldetag: 05.07.2011
(51) Int. Cl.: A61M 11/02, A61M 11/06, A61M 15/00, B05B 1/00, B05B 11/00

(54) **FILTERSYSTEM FÜR DEN EINSATZ IN MEDIZINISCHEN GERÄTEN**
FILTER SYSTEM FOR USE IN MEDICAL DEVICES
SYSTÈME DE FILTRE CONÇU POUR ÊTRE UTILISÉ DANS DES APPAREILS MÉDICAUX

(30) Priorität: 16.07.2010 EP 10169883; 16.07.2010 EP 10169878
(43) Veröffentlichungstag der Anmeldung: 22.05.2013
(73) Patentinhaber: Boehringer Ingelheim International GmbH, 55216 Ingelheim am Rhein (DE)
(72) Erfinder: HAUSMANN, Matthias, 55216 Ingelheim am Rhein (DE); SCHUY, Steffen, 55216 Ingelheim am Rhein (DE); EICHER, Joachim, 55216 Ingelheim am Rhein (DE); GESER, Johannes, 55216 Ingelheim am Rhein (DE); MEISENHEIMER, Martin, 55216 Ingelheim am Rhein (DE); WITTE, Florian, 55216 Ingelheim am Rhein (DE)
(74) Vertreter: Simon, Elke Anna Maria
(86) Internationale Anmeldenummer: PCT/EP2011/061288
(87) Internationale Veröffentlichungsnummer: WO 2012/007315

(56) Entgegenhaltungen:
- EP-A1- 0 891 712
- EP-A1- 2 044 967
- EP-A1- 2 275 160
- WO-A1-2005/000476

## Beschreibung

Die vorliegende Erfindung betrifft Geräte zum Verabreichen von flüssigen, medizinischen Formulierungen, in denen die Flüssigkeit durch enge Strömungskanäle geleitet wird. Insbesondere bezieht sich die Erfindung auf kleinere portable Geräte wie beispielsweise Dosisspender, Zerstäuber oder Injektoren, wie sie für das Abmessen, die Inhalation oder Injektion von flüssigen Arzneimittelformulierungen verwendet werden. Insbesondere bezieht sich die Erfindung auf einen Zerstäuber zur Vernebelung ethanolischer und/oder wässriger medizinischer Formulierungen mit einem in einen Strömungskanal vor einer Austrittsdüse eingesetzten Grobfilter zum Abscheiden von Partikeln.

Aus dem Stand der Technik ist eine Vielzahl an medizinischen Geräten und auch konkret Zerstäubern bekannt, die mit einer Flüssigkeit betrieben werden. Bei den meisten ist die Flüssigkeit in Vorratsgefäßen oder Behältern abgefüllt, die mehrere Einheiten zur Verabreichung mit dem Gerät beinhalten, so dass die Strömungskanäle, Dosiereinheiten, Ausbringungsöffnungen wie z.B. Zerstäubungsdüsen des Geräts mehrfach verwendet werden. Im Bereich der Zerstäuber trifft dies beispielsweise sowohl auf Treibgasenthaltenden Metered Dose Inhaler (MDIs), in denen bei Betätigung eines Dosierventils das Treibgas die Formulierung aus einem Vorratsbehälter immer wieder durch die gleiche Düse austreibt, als auch auf rein mechanische Zerstäuber wie beispielsweise handelsübliche Nasenspraysysteme oder Fingerpumpsysteme zu.
Alle diese Systeme sind potentiell anfällig gegen Verlegung von Strömungskanälen oder Austragsdüsen. Solche Verlegungen können beispielsweise durch Abriebpartikel beim Gebrauch oder bei der Montage des Systems oder durch Agglomeration von Formulierungsbestandsteilen entstehen. Das Entstehen dieser Verlegungen ist abhängig von der Zusammensetzung der Formulierung, den Löslichkeiten ihrer Bestandteile, dem Wechselwirkungspotential der Bestandteile untereinander und mit Gerätebauteilen im Fluidkontakt und von dem Verhalten der Formulierung bei unterschiedlichen klimatischen Bedingungen insbesondere bei verschiedenen Temperaturen.

Die EP 0 521 061 B1 offenbart ein Dosiergerät in Form eines rein mechanischen Zerstäubers, in dem eine abgemessene Menge von flüssigem Medikament in eine Druckkammer geleitet wird, aus der das flüssige Medikament mittels Kolbendruck durch einen Zerstäuberkopf ausgebracht wird. Im Einlasskanals des Zerstäuberkopfes ist ein einzelner Filter - beispielhaft in Form eines Maschengitters - angeordnet, um Partikel auf dem Weg zur Austrittsdüse abzufangen.

Ein Zerstäuber, mit dem flüssige Arzneimittelformulierungen zur Inhalation aus einem mehrere Einheiten der Formulierung enthaltenden Behälter zerstäubt werden, wird seit Jahren unter dem Namen "Respimat®" von der Boehringer Ingelheim KG angeboten. Dieser rein mechanische, miniaturisierte Hochdruckzerstäuber ist in der WO97/12687A1 und WO09/047173A2 dargestellt. Mit diesem Zerstäuber wird eine flüssige Arzneimittelformulierung aus einem in den Zerstäuber eingesetzten starren Behälter mit Innenbeutel, wie in der WO00/49988A2 offenbart, mittels einer durch ein Schraubgetriebe angetriebenen Kolbenpumpe aus dem Innenbeutel gefördert und mittels eines federgetriebenen Druckerzeugers in vorbestimmter Menge durch eine mikrostrukturierte Düse zu einem lungengängigen Aerosol zerstäubt. Die Zerstäubung durch die Düse beruht auf der Impaktion von zwei mikroskopischen Flüssigkeitsstrahlen mit hoher Geschwindigkeit, wodurch sich ein feiner Nebel bildet. Details möglicher Mikrostrukturen für in den Zerstäubern eingesetzte Austragsdüse werden in den Schriften WO94/07607A1, WO99/16530A1 und WO05/000476A1 offenbart. In der WO09/047173A2 wird der Strömungsweg der Arzneimittelzubereitung aus dem Behälter durch ein Förderrohr in die Druckkammer und daraus über einen Vorfilter aus Kunststoff in die mikrostrukturierte Austragsdüse beschrieben. Nach dem Größenausschlußprinzip werden Partikel in der Größenordnung der Austrittskanäle der Düse im Strömungsweg vor der Düse abgefangen.

In der Schrift US6837866B1 ist der Einsatz eines Filters in einem nadellosen Injektionssystem beschrieben. Die aktive Reagenz wird hierbei über einen Druckgasstoß ausgebracht, der schockwellenartig die Membranen aufbricht, zwischen denen sich die aktive Reagenz befindet. Der hier eingesetzte Filter aus gestapelten metallischen Netzen mit einer abschließenden Keramikschicht dient hierbei nicht zur Filterung der aktiven Reagenz selbst, sondern rein der Filterung des Druckgases und vor allem seiner Abkühlung (bei der Erzeugung des Druckgasstoßes wird mit einem pyrotechnischen Generator gearbeitet und die hierbei erzeugten Temperaturen sollen nicht in direkten Kontakt mit der aktiven Reagenz kommen).

Die EP 08917121 A1 offenbart ein Gerät zur Ausgabe einer sterilen Flüssigkeit wie z.B. in Form von Augentropfen. Das Gerät besteht aus einem Behälter zur Bevorratung der Flüssigkeit und einer Düsenanordnung, welche direkt am Behälter angeordnet ist. Integriert im Gerät ist ein antimikrobielles Element zur Unterbindung von mikrobiologischer Kontamination bei wiederholter Anwendung des Geräts. In einer Ausführungsform beinhaltet das antimikrobielle Element eine serielle Anordnung von Filtern, die so mit der Düsenanordnung verbunden sind, dass die Flüssigkeit- (und Luft-)-Strömungen durch die Filter geleitet werden. Die Filter bestehen aus einem Substrat auf dessen Oberfläche und in dessen Poren ein antimikrobielle Agens aufgebracht ist. Das antimikrobielle Agens kann ein metallisches oder nicht-metallisches Material mit anti-bakteriellen, anti-viralen und/oder antimykotischen Eigenschaften oder einer Kombination davon sein. Hierbei sind die Oberflächen und Poren der Filter mit verschiedenen antimikrobiellen Materialien beschichtet. In einer Ausführungsform weist das Abgabegerät einen Vorfilter z.B. aus Glasfasern, synthetischen Polymerfasern, hydrophilen Polypropylenfasern, Nylon oder Zellulosefasern auf, der eine Barriere für partikuläres Material darstellt. Die Düsenanordnung beinhaltet eine Scheibe mit einer Vielzahl von konzentrischen Kanälen, die radial mit einander und mit einer zentralen Durchführung verbundenen sind. Flüssigkeit, die aus dem Behälter kommt, passiert flächig die Filter und wird auf der Scheibe verteilt, wo sie in die zentrale Durchführung fließt, um von dort als einzelner Tropfen oder Flüssigkeitsstrom abgegeben zu werden.

Ausgangspunkt für die hier dargestellte Entwicklung ist die bisherige in medizinische Handgeräte wie dem in der WO09/047173A2 beschriebener Zerstäuber integrierte Filterung von Flüssigkeiten.

Der vorliegenden Erfindung liegt die Aufgabe zugrunde, ein gegenüber dem Stand der Technik verbessertes Gerät zum Verabreichen von flüssigen medizinischen Formulierungen - insbesondere ein Handgerät wie einen Zerstäuber oder Injektor - anzugeben, das ein insbesondere für kleine Strömungsquerschnitte und auf die Abscheidung unterschiedlichster Partikel ausgelegtes Filtersystem beinhaltet. Die mit diesem Filtersystem ausgestatteten Geräte sollen möglichst unabhängig von der späteren Verwendung, d.h. insbesondere unabhängig von der Wahl des Lösungsmittels der Formulierung, den Kompatibilitätseigenschaften und den klimatischen Bedingungen sein. Das Filtersystem und das dazugehörige Montagekonzept sollen für die Massenfertigung geeignet sein.
Insbesondere soll ein Filtersystem angegeben werden bei dem eine wirksame Filtration der flüssigen medizinischen Formulierung stattfindet. Besonders bevorzugt sollen durch das Filtersystem auch kleinste Rückstände mit einem Durchmesser kleiner 1 µm aus der Inhalationslösung oder Abriebpartikel filtriert werden. Solche kleinste Partikel werden teilweise nicht von typischen Größenausschussfiltern erfasst, die zuverlässig Partikel mit Durchmesser von mindestens im Mikrometerbereich ausfiltern.

Die genannte Aufgabe wird erfindungsgemäß gelöst durch ein Gerät zum Verabreichen einer flüssigen medizinischen Formulierung, die sich in einem in das Gerät eingesetzten Behälter befindet und durch mindestens eine, vorzugsweise zwei Düsenöffnungen aus dem Gerät ausgebracht wird, wobei die flüssige medizinische Formulierung vor Durchströmen der mindestens einen Düsenöffnung durch einen Feinstfilter strömt und wobei im Inneren des Geräts im Strömungsweg der Flüssigkeit ein Vorfilter vor dem Feinstfilter angeordnet ist. Der Feinstfilter und vorzugsweise auch die mindestens eine Düsenöffnung werden von einem mikrostrukturierten Bauteil gebildet. Das Gerät ist dadurch gekennzeichnet, dass im Inneren des Geräts im Strömungsweg der Flüssigkeit ein Feinfilter zusätzlich zwischen Vorfilter und dem mikrostrukturierten Bauteil angeordnet ist, wobei der Feinfilter zum Vorfilter verschiedenartig ist, so dass an Vorfilter und Feinfilter jeweils Partikel unterschiedlicher Größe oder Art vor Eintritt der Formulierung in das mikro strukturierte Bauteil abgeschieden werden.

Das erfindungsgemäße Gerät wird im Anspruch 1 definiert.

Des weiteren wird die erfindungsgemäße Aufgabe durch ein Verfahren zur Montage eines Geräts gelöst, das eine Druckkammer und zwischen der Druckkammer und Düsenkanälen angeordnete Filter beinhaltet, wobei die Filter durch die spätere Druckkammer hindurch in ein Zentralteil eingeführt werden und die Einführöffnung durch den Kolben eines Druckerzeugers oder durch ein insbesondere als Hohlkolben gestaltete Verbindungselement zur späteren Entnahme von Flüssigkeit aus einem Behälter verschlossen wird.

Vorteilhafte Weiterbildungen werden im Folgenden und im Detail anhand der Figuren beschrieben.

Ein Merkmal der vorliegenden Erfindung ist, dass die Filter unterschiedliche Porengrößen aufweisen und so angeordnet sind, dass die Porengrößen im Strömungsverlauf kleiner werden. Vorteil dieser Erfindung ist, dass bei so einem System aus hintereinander angeordneten Filtern die Partikelabscheidung auf mehrere Stellen verteilt wird und es bei hohem Partikelaufkommen nicht zu einer Komplettverlegung des Filters und somit des Geräts insgesamt kommt. Dies ist insbesondere für solche Geräte wie die hier vorzugsweise betrachteten Handgeräte bedeutsam, in denen die Flüssigkeit auch schon vor der Ausbringung durch Strömungskanäle von begrenztem Durchmesser geleitet wird und in denen entweder aufgrund der zur Verfügung stehenden Einbauräume oder aufgrund von strömungstechnischen Bedingungen keine großflächigen Filter eingebaut werden können. In solchen "engen" Einbausituationen ist die Abscheidungskapazität eines einzelnen Filters aufgrund seiner relativ kleinen Querschnittsfläche begrenzt.

Ein weiteres Merkmal der vorliegenden Erfindung ist, dass die Filter aus unterschiedlichen Materialien bestehen. Auf diese Weise wird der durch die Porengröße der Filter bestimmte Filtereffekt unterstützt durch die unterschiedlichen Adsorptionseigenschaften der Filtermaterialien: Je nach Material und Oberflächenbeschaffenheit der Filter bleiben Partikel zusätzlich zur Größenabscheidung am Filter haften. Bei der Verwendung von unterschiedlichen Filtermaterialien werden auf diese Weise auch verschiedene Arten von Partikeln über Adsorption abgeschieden.

Ein weiteres Merkmal der vorliegenden Erfindung ist, dass ein Adsorptionsfilter in dem Strömungsweg zwischen dem Grobfilter und der Austrittsdüse angeordnet ist. Der Adsorptionsfilter trennt Nanopartikel aus der Flüssigkeit auf der Basis intermolekularer Wechselwirkungen wie etwa Elektrostatik, van der Waals Kräften oder der Bildung von Wasserstoffbrückenbindungen ab. Der, insbesondere aus einem Kunststoff wie beispielsweise Polyethylen gefertigte, Vorfilter - der hier auch als Grobfilter bezeichnet werden kann - scheidet im Strömungsweg zuerst Teilchen bis runter zu einer Größengrößenordnung von etwa 9 µm aus der flüssigen medizinischen Formulierung ab und der nachgeschaltete Adsorptionsfilter filtriert anschließend Nano-Partikel, insbesondere bis zu 5 nm Korngröße aus der flüssigen medizinischen Formulierung. Bei hohen Durchflussraten können solche Nanopartikel aufgrund ihres kleinen Durchmessers nicht mittels herkömmlicher Größenausschlußfilter abfiltriert werden. Durch die Abscheidung der Nanopartikel wird verhindert, dass sich solche Nanopartikel an den Auslässen der Austrittsdüse anlagern und somit zu einer Beeinträchtigung der Zerstäubung, bzw. insbesondere bei der Verwendung von Zweistrahldüsen zu einer Richtungsabweichung der Flüssigkeitsstrahlen führen können.

Vorzugsweise wird ein Adsorptionsfilter verwendet, der u.a. eine große Durchflussrate garantiert. Besonders bevorzugt wird hierbei ein Adsorptionsfilter mit einer großen insbesondere "inneren" Oberfläche verwendet, so dass sich möglichst viele Nanopartikel an ihr anlagern können. Insgesamt wird durch die Anordnung des Adsorptionsfilters im Strömungsweg die Zuverlässigkeit des Zerstäubers hinsichtlich der Vernebelung der flüssigen medizinischen Formulierung gesteigert und eine wirksame Filtration der Flüssige medizinische Formulierung zur Vermeidung einer Beeinträchtigung der Austrittsdüse findet statt.

Die Erfindung liegt darin, dass einer der Filter aus Titan in Form eines Sintermetalls besteht.

Auf diese Weise erhält man Filtermaterialien mit guten chemischen Verträglichkeiten insbesondere zu Formulierungen, die auf organischen Lösungsmitteln wie beispielsweise Ethanol basieren und/ oder insbesondere saure pH-Bereiche aufweisen. Gesinterte Strukturen können gut in Strukturen mit kleinen Querschnitten eingepresst werden. Ein Filterbauteil aus Sintermetall ist mechanisch stabil und zeigt gute Einbaueigenschaften, so dass es insbesondere bei Einbau in ein Bauteil aus vergleichsweise weicherem Material bzw. Kunststoff im Einbauzustand nicht zur Bildung von Bypässen an den Seitenwänden kommt. Es hat sich gezeigt, dass durch die Verwendung von Sintermetall aus spratzig fraktionierten Metallpulvern Filter mit besonders kleinen Porengrößen geschaffen werden können, so dass kleinere Partikel herausgefiltert werden ist als bei den bisher im Stand der Technik bei Zerstäubern verwendeten Partikelfiltern.

Bevorzugt ist der Feinfilter ein solcher Filter aus Metall oder ein Adsorptionsfilter.

Besonders bevorzugt weist das Filtersystem zusätzlich zu einem Adsorptionsfilter mehrere Filter auf, mit denen im Größenausschlußverfahren weitere Partikel aus der vernebelten flüssigen medizinischen Formulierung herausgefiltert werden. Insbesondere weist das Filtersystem einen Grob- oder Vorfilter, einen Feinfilter aus Metall und einen Adsorptionsfilter auf. Der Feinfilter kann wahlweise zwischen dem Vorfilter und dem Adsorptionsfilter oder zwischen dem Adsorptionsfilter und dem die Düse bildenden mikrostrukturiertem Bauteil bzw. dem Feinstfilter angeordnet sein.

Gemäß eines weiteren Merkmals der Erfindung ist der hier verwendete Adsorptionsfilter aus Glas-, Zellulose-, Kohle- oder Polymerfasern gefertigt und wird vor seiner Verwendung einer Funktionalisierung der Oberfläche unterzogen. Somit ist die Trägerfaser funkionalisiert, sofern das Trägermaterial nicht selbst, möglicherweise auf Grund seiner intrinsischen Oberflächeneigenschaften, als adsorptive Faser verwendet werden kann, wie zum Beispiel eine Glasfaser (pH Wert abhängige Oberflächenladungen und freie Hydroxygruppen (OH-Gruppen) zur Wasserstoffbrückenbildung). Eine mögliche Funktionalisierung wird beispielsweise mittels quartären Ammoniumgruppen durchgeführt, was zur Ausbildung von Oberflächenladungen führt. Wird auf diese Weise die Oberfläche des Adsorptionsfilters positiv aufgeladen, können sich typische negativ geladene Partikel, vorzugsweise glasartige Substanzen, anlagern.

Besonders bevorzugt wird die Möglichkeit, die Oberflächen des Adsorptionsfilters mittels Aluminiumoxiden zu funktionalisieren. Hierdurch bilden sich an der Oberfläche zwei verschiedene Funktionalitäten, über die Partikel angelagert werden können: sowohl eine positive Aufladung der Oberfläche als auch Hydroxygruppen des Aluminiumoxidhydroxid (AlO(OH)), an denen sich über die Bildung von Wasserstoffbrückenbindungen zusätzlich Partikel anlagern können. Bei Versuchen mit den erfindungsgemäßen Filtern zeigten Adsorptionsfilter mit einer mittels Aluminiumoxids funktionalisierten Oberfläche gute Adsortionseigenschaften bei Verwendung von flüssigen medizinischen Formulierungen mit pH-Werten im Bereich von pH 3,5 bis 9. Bei Verwendung von sauren Flüssigkeiten ist die Protonierung der mittels Aluminiumoxid funktionalisierten Oberfläche und somit ihre positive Aufladung verstärkt und bei basischen Flüssigkeiten ist die Bildung von Wasserstoffbrücken durch eine erhöhte Flächendichte von freien OH-Gruppen verstärkt. Hierdurch lässt sich bei Verwendung von mittels Aluminiumoxid funktionalisierten Adsorptionsfiltern insbesondere eine Agglomeration von Silika-Nanopartikeln mit einer Größe << 100 nm am Auslass der Austrittsdüse des Zerstäubers verhindern. Silikate sind Füllstoffe in typischen elastomeren Dichtungen, wie sie auch in Zerstäubern verwendet werden. Daher können solche Silika-Nanopartikel z.B. als Abrieb während der Benutzung des Zerstäubers entstehen, wie überraschenderweise festgestellt werden konnte. Bekannte gemäß Adsorptionsprinzipien wirkende Filter sind unter verschiedenen Handelsnamen auf dem Markt erhältlich, wobei NanoCeram®, Fa. Argonide Advance Filtration Technologies, Sanford, FL, USA, als Filter mit einem elektropositiven Filtermaterial und alternativ ZetaPlus®, Fa. 3M Purification Inc., St. Paul, MN, USA, oder Disruptor™, Fa. Ahlstrom Corporation, Helsinki, Finnland, genannt sein sollen. Darüber hinaus sind auch handelsübliche Glasfaserfilter mit entsprechend hoher freier Oberfläche als Adsorptionsbasierte Filter für verdünnte Dispersionen dienlich (z.B. Glasfaserfilter von Fa. GE Whatman GF Series). Bei den vorgenannten Filtermaterialien handelt es sich lediglich um Beispiele, wobei weitere auf Adsorption basierte Filtermaterialien von ihrer Verwendung nicht ausgeschlossen sind.
Unter dem Begriff Filterkapazität ist bei Filterungen nach dem Größenausschussprinzip ein Maß für die Partikelmenge zu verstehen, die vom Filter beim gleichzeitigen Durchlass von Flüssigkeit noch abgefangen werden kann. Die Filterschwelle ist in diesem Zusammenhang ein Maß für die Größe der Partikel, die durch den Filter zurückgehalten werden. Diese Filterschwelle stellt den Trenngrad der Filterung dar, d.h. für die zur Filterschwelle angegebene Porengröße ist entsprechend der verwendeten Normen jeweils eine definierte hohe Wahrscheinlichkeit, z.B. 90%, gegeben, dass Partikel von mindestens dieser Größe am Filter abgeschieden werden.
Die Filterschwelle eines Filters wird bestimmt durch seine Porengröße. Die Porengröße eines Filters kann beschrieben werden durch die rechnerische Größe des Porendurchmessers, dessen Verteilung mittels der Washburn-Gleichung (E.W. Washburn, Proc. Natl. Acad. Sci. USA, 7, 115 (1921)) aus Messdaten ermittelt werden kann, die mittels etablierter Kapillardruck-Messmethoden wie insbesondere der Quecksilber-Porosimetrie gewonnen werden, wie sie beispielsweise in der deutschen DIN-Norm 66133 beschrieben wird.

Bei den hier dargestellten Geräten zum Verabreichen von flüssigen medizinischen Formulierungen werden bevorzugt Handgeräte wie Zerstäuber oder Injektoren betrachtet, mit denen Flüssigkeiten in definierten Volumina ausgebracht, d.h. zerstäubt oder eingespritzt werden.
Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen. Unter dem Begriff "medizinische Formulierung" oder "Arzneimittelformulierung" sind bei der vorliegenden Erfindung über Medikamente und Wirkstoffe enthaltende Formulierungen hinaus auch Therapeutica oder dergleichen, insbesondere also jede Art von Mitteln zur Inhalation oder sonstigen Anwendung zu verstehen.

Die einzelnen Merkmale der vorliegenden Erfindung können unabhängig voneinander genutzt oder miteinander kombiniert werden.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
- Fig. 1: einen schematischen Schnitt eines Zerstäubers im ungespannten Zustand,
- Fig. 2: einen schematischen, um 90° gegenüber Fig.1 gedrehten Schnitt des Zerstäubers aus Fig. 1 im gespannten Zustand,
- Fig. 3: einen schematischen Schnitt durch die Zerstäuber-Bauteile Düse, Filtertechnik, Pumpenkammer und Verbindungselement zum Behälter.
- Fig. 4: einen Schnitt durch ein mikrostrukturiertes Bauteil bzw. eine schematische Draufsicht eines die Mikrostruktur mit Düse zeigenden Teils des mikrostrukturierten Bauteils zum Einbau in einen Zerstäuber.
- Fig. 5: die Seitenansicht eines seitlich gekippten einzelnen Filterelements zum Einbau in ein medizinisches Handgerät,
- Fig. 6: einen schematischen Schnitt durch die Düsenbaugruppe des Zerstäubers mit vorgelagertem Filtersystem entsprechend einer Ausführungsform mit Adsorptionsfilter,
- Fig. 7: einen schematischen Schnitt durch die Düsenbaugruppe des Zerstäubers mit vorgelagertem Filtersystem entsprechend einer weiteren Ausführungsform mit Adsorptionsfilter,
- Fig. 8: einen schematischen Schnitt durch die Düsenbaugruppe des Zerstäubers mit vorgelagertem Filtersystem entsprechend wiederum einer weiteren Ausführungsform mit Adsorptionsfilter.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

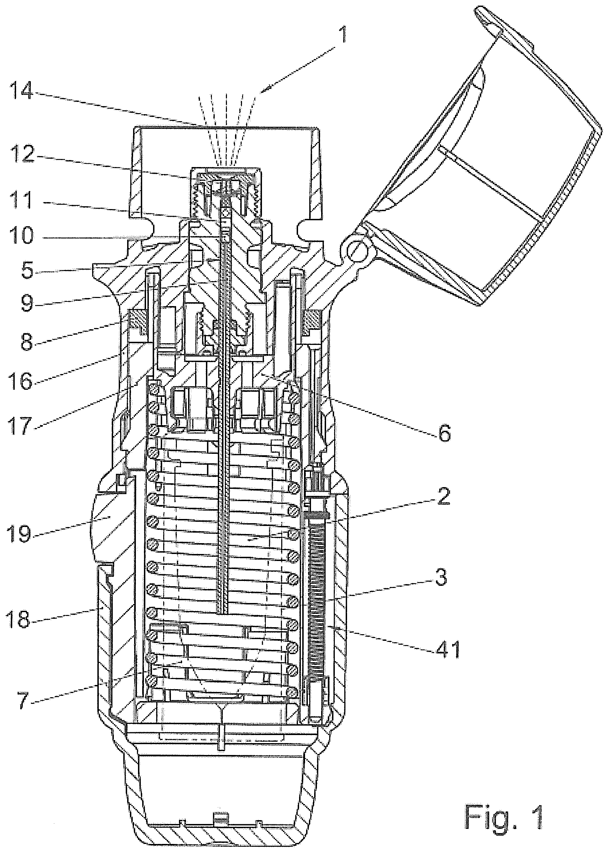

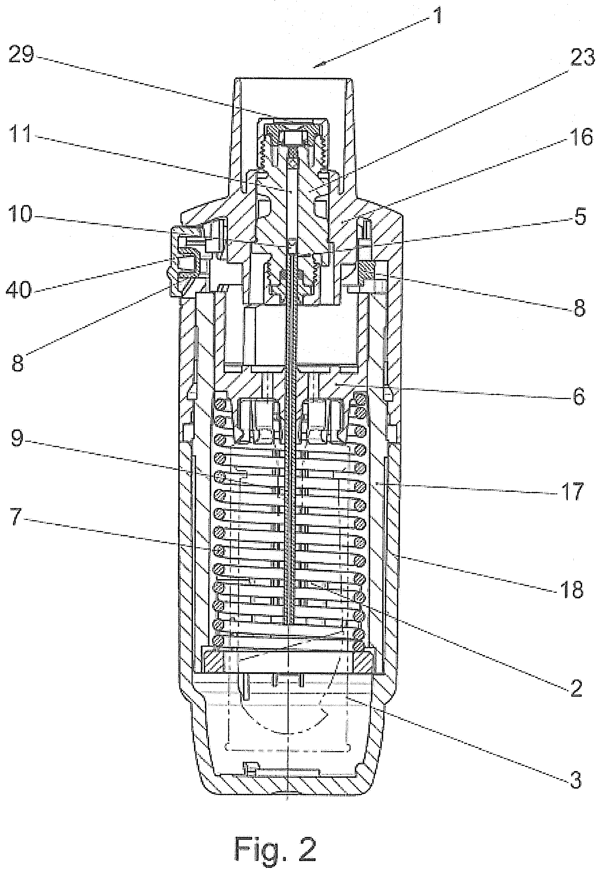

Fig. 1 und 2 zeigen in einer schematischen Darstellung ein handbetätigtes medizinisches Gerät, in dem das vorschlagsgemäße Filterkonzept eingesetzt werden kann. Bei dem Gerät aus Fig. 1 und 2 handelt es sich um einen treibgasfreien Zerstäuber (1), der pro Betätigungszyklus die jeweilig vorbestimmte Menge einer Flüssigkeit (2) bzw. einer flüssigen medizinischen Formulierung als vorzugsweise lungengängiges bzw. inhalierfähiges Aerosol (14) ausgibt. Dieses Aerosol (14) mit Tröpfchen mit aerodynamischen Durchmessern von vorzugsweise 0,5 bis 10 Mikrometern, insbesondere 0,5 bis 5 Mikrometer kann von einem nicht dargestellten Benutzer eingeatmet werden. Zur Zerstäubung wird hierbei eine geeignete Düse verwendet, die im Darstellungsbeispiel in ein mikrostrukturiertes Bauteil (12) integriet ist. Tauscht man die der Zerstäubung dienende Düse dieses Geräts gegen den Kopf eines Flüssigkeitsspenders oder eine Injektionsdüse bzw. eine Kanüle oder sonstige Einspritzvorrichtung, so bleiben alle Düsenunabhängigen Funktionsprinzipien unverändert, so dass die im Nachfolgenden geschilderten Zusammenhänge ganz analog für Injektoren oder andere Flüssigkeitsausbringende Systeme ihre Gültigkeit haben, selbst wenn der Einfachheit halber nur Zerstäuber genannt werden.

Beim Betrieb des Zerstäubers wird unterschieden zwischen dem ungespannten Zustand mit unbefülltem Dosiervolumen in der Druckkammer (11) (Fig. 1) und dem gespannten Zustand mit befüllter Druckkammer (11) (Fig. 2).

Beim Spannen des Zerstäubers (1) wird sein Gehäuseoberteil (16) relativ zum Gehäuseinnenteil (17) und Gehäuseunterteil (18) um einen festen Drehwinkel wie z.B. 180° gedreht. Mittels eines innen angeordneten Schraubgetriebes wird durch die Relativdrehung eine Kolbenpumpe angetrieben, so dass eine vorbestimmte, ggf. einstellbare Menge der Flüssigkeit (2) aus dem Behälter (3) in die Druckkammer gefördert und gleichzeitig die Antriebsfeder (7) des Druckerzeugers (5) gespannt wird (Endzustand des Spannvorgangs ist in Fig. 2 dargestellt). Beim Auslösen des Zerstäubers (1), d.h. bei Betätigung eines Sperrings (8) über eine Taste (40) wird die in der Antriebsfeder (7) gespeicherte Energie des Druckerzeugers (5) freigesetzt: Der zuvor zur Flüssigkeitsförderung benutzte Hohlkolben (9) drückt nun mit geschlossenem Rückschlagventil (10) in die Druckkammer (11), so dass die durch die Hubbewegung des Hohlkolben (9) vorbestimmte Flüssigkeitsmenge von dort durch die Düse ausgebracht wird. Das Gerät befindet sich nun wieder im entspannten Zustand (Fig. 1).

Im Darstellungsbeispiel ist der Hohlkolben (9) fest mit einer zum Druckerzeuger (5) gehörenden Halterung (6) für den Behälter (3) verbunden - beispielsweise angespritzt, verklebt oder verschnappt. Der Behälter (3) wird über die Halterung (6) insbesondere klemmend oder rastend so in dem Zerstäuber (1) fixiert, dass der Hohlkolben (9) in den Fluidraum des Behälters (3) eintaucht und/oder fluidisch mit der Flüssigkeit (2) im Behälter (3) verbunden wird und sie über den Hohlkolben angesaugt werden kann. Der Behälter kann wahlweise austauschbar sein. Das Gerätegehäuse kann zu diesem Zweck so gestaltet sein, dass es aufgemacht oder teilweise abgenommen werden kann (z.B. in Form eines kappenartigen Gehäuseunterteils wie in WO07/128381A1 offenbart).
Der Behälter (3), der in den mit einem Dosisindikator oder einem Zählwerk (41) ausgestatteten Zerstäuber (1) eingesetzt wird, ist für die Entnahme mehrerer Dosiseinheiten ausgelegt. Dazu muss er so beschaffen sein, dass der Innendruck auch bei Flüssigkeitsentnahme im wesentlichen unverändert bleibt, so dass beim Ansaugen immer die gleiche Menge Flüssigkeit (2) entnommen wird. Hierzu kann prinzipiell sowohl ein Behälter (3) mit starrer Behälterwand, dessen Innendruck über eine Belüftung konstant gehalten wird und wie er beispielsweise in der WO06/136426A1 beschrieben wird, als auch ein Behälter (3) mit einer flexiblen Wand verwendet werden, die sich bei Flüssigkeitsentnahme zumindest teilweise derart ins Behälterinnere verschiebt, dass durch Verkleinerung des Innenvolumens der Innendruck konstant gehalten wird. Bevorzugt werden hierbei Behälter (3), in denen die flexible Wand durch einen Beutel gebildet wird, der im Wesentlichen verformbar, zusammendrückbar und/oder zusammenziehbar ausgebildet ist. Solche Behälter werden in verschiedenen Ausführungsformen in den Schriften WO00/49988A2, WO01/076849A1, WO99/43571A1, WO09/115200A1 und WO09/103510A1 beschrieben. Besonders bevorzugt besteht der Behälter aus einem flexiblen, mehrschichtigen, unten geschlossenen Folienbeutel, der im oberen Bereich direkt mit einem Halt gebenden Flansch vorzugsweise aus Kunststoff verbunden ist, einer daran angeschweißten Behälterkappe zur Anbindung an die Halterung (6) des Zerstäubers (1), einer äußeren Schutzhülse und einem Kopfsiegel (Details siehe WO99/43571A1 und WO09/115200A1).

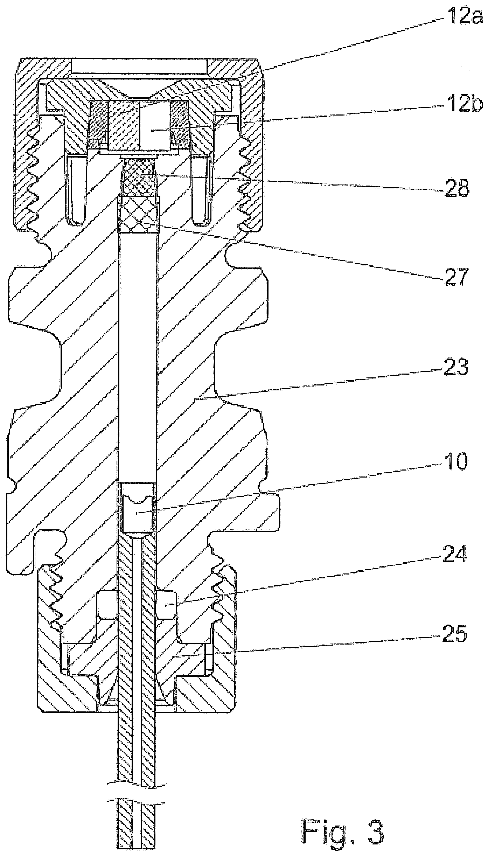

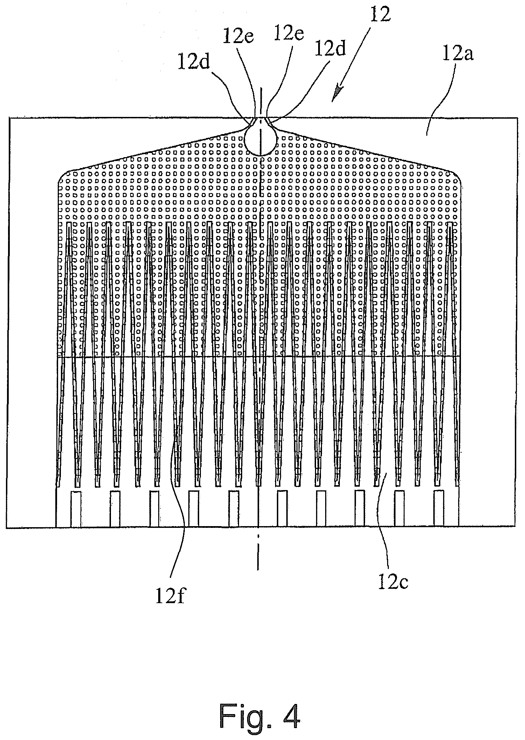

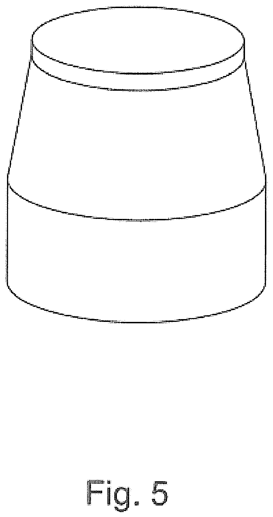

Fig. 3 zeigt in schematischer Darstellung die Druckkammer (11) des Zerstäubers (1), der in der dargestellten Ausführungsform sowohl für die Zerstäubung von wässrigen flüssigen Formulierungen als insbesondere auch für die Zerstäubung von alkoholischen flüssigen Formulierungen geeignet ist. In die Druckkammer (11) taucht behälterseitig der zum Kolbenpumpensystem gehörende Hohlkolben (9) ein. Der Hohlkolben (9) ist zugleich das Verbindungselement zwischen Druckkammer (11) und dem Inneren des Behälters (3). Wird der Hohlkolben (9) im Rahmen des Spannvorgangs teilweise aus der Druckkammer (11) herausgezogen, entsteht dort ein Unterdruck, durch den über das in dieser Situation offenen Rückschlagventil (10) im Hohlkolben (9) Flüssigkeit (2) aus dem Behälter (3) in die Druckkammer (11) leitet. Schnellt der Hohlkolben (9) beim Auslösen des Zerstäubers (1) in die Druckkammer (11) hinein, ist das Rückschlagventil (11) durch Anliegen seiner Dichtflächen am Sitz im Hohlkolben geschlossen und die Flüssigkeit in der Druckkammer (11) wird unter Druck durch ein Filtersystem und die Düse ausgetrieben. Hohlkolben (9) und Druckkammer (11) sind durch eine elastomere Dichtung (24) abgedichtet, die insbesondere die Form eines O-Rings hat und sich in der Führungsröhre des Kolbens nahe dessen Eintritt in die Druckkammer (11) befindet; die geometrische Einbausituation dieser Dichtung (24), die vorzugsweise mittels eines Stützrings (25) verpresst wird, entspricht beispielsweise der in der WO07/051536A1 beschriebenen.
Im Flüssigkeitsauslassbereich der Druckkammer (11) befindet sich ein Filtersystem, das dem vorzugsweise mikrostrukturierten Bauteil (12) vorangeht, in das die Düse integriert ist. Das hier dargestellte neuartige Filtersystem besteht aus mehreren, hinter einander angeordneten Filterbauteilen, die sich insbesondere durch die angewendete Filtertechnik unterscheiden. Vorzugsweise sind die Filterschwellen der einzelnen Filterbauteile derart bemessen, dass jeder Filter nach dem Größenausschluss-Prinzip kleinere Partikel durchlässt als sein Vorgänger. Über die Kombination verschiedener Filtertechniken und der Anordnung von Filtern mit sukzessiv wachsendem Trenngrad, bzw. sukzessiv kleiner werdenden Porengrößen wird insgesamt eine höhere Filterkapazität, d.h. die Abscheidung von größeren Partikelmengen ohne komplette Filterverlegung, und eine gründlichere Filterung erreicht. Der im Strömungsweg zuerst eingebaute Filter mit dem größten Porendurchmesser fängt nur die großen Partikel, der nächste Filter mit kleinerem Porendurchmesser fängt kleinere Partikel auf und so weiter. Auf diese Weise wird ein feinporiger Filter nicht direkt so durch große Partikel verlegt, dass er überhaupt keine Flüssigkeit mehr durch lassen kann.
Zusätzlich zum Auffangen fester Partikel einer bestimmten Größe kann ein Filter gegebenenfalls zusätzliches Material über Adsorption abfangen. Verwendet man nun Filter unterschiedlicher Beschaffenheiten und unterschiedlicher Materialien so wird sich diese zusätzliche Adsorption von Filter zu Filter unterscheiden. Durch die Kombination von verschiedenen Filtertechniken können dementsprechend auch mehr Partikel und insbesondere auch unter Druck verformbare Partikel dank der verschiedenen Adsorptionseffekte abgefangen werden.
Die Notwendigkeit zur Partikelfilterung ergibt sich bei der Zerstäubertechnik insbesondere daraus, dass zur Sicherung der Funktionsfähigkeit die Zerstäuberdüse von Verlegungen frei gehalten werden muss. Zur Erzeugung von inhalierfähigen Aerosolen benötigen die meisten Zerstäubungskonzepte sehr klein dimensionierte Düsenstrukturen, die häufig mittels so genannter Mikrosystem-Technologien wie lithographischer Herstellungsmethoden aus der Halbleiter-Fertigung oder Funken-Erosion oder Laserbohrtechniken hergestellt werden.
Im Falle des Ausführungsbeispiels betragen die Abmessungen der Düsenkanäle (12d) des favorisierten mikrostrukturierten Bauteils (12) nur wenige Mikrometer. Bevorzugt haben die Düsenkanäle (12d) ein rechteckiges Profil mit Kantenlängen von 2 bis 10 Mikrometern. Ein für den Einsatz im Ausführungsbeispiel verwendbares mikrostrukturiertes Bauteil (12) ist in Fig. 4 dargestellt. Im Beispiel beruht die Zerstäubung der Flüssigkeit mit dem Zerstäuber vorzugsweise auf der Impaktion von zwei mikroskopischen Flüssigkeitsstrahlen mit hoher Geschwindigkeit: Aus den vorzugsweise zwei Düsenkanälen (12d) bzw. den zugehörigen Düsenöffnungen (12e) treten Flüssigkeitsstrahlen derart gerichtet aus, dass sie in einem definierten Winkel aufeinander treffen und durch die beim Aufprall wirkenden Kräfte vernebelt werden. Lagern sich bei Betrieb des Geräts in diesen Düsenkanälen (12d) Partikel an, so können die Flüssigkeitsstrahlen gegebenenfalls abgelenkt werden, so dass die Impaktion und somit die Zerstäubung nicht mehr vollständig ist bzw. im Extremfall gar nicht zustande kommt. Aus diesem Grund sollten die Partikel bereits möglichst vollständig aus der Flüssigkeit (2) herausgefiltert werden, bevor diese in die Düsenkanäle (12d) einströmt.
Partikel können innerhalb des Zerstäubers auf unterschiedlichste Weise entstehen: durch Agglomeration oder Ausflockung in der flüssigen medizinischen Formulierung, durch Prozessschritte bei der Montage des Zerstäubers und durch Abrieb an im Gerät gegeneinander bewegten Bauteilen, z.B. im Bereich von dynamischen Dichtungen.
Das Zentralteil (23) bildet die seitliche Begrenzung der Druckkammer (11), den Flüssigkeitseinlass in Form der Durchführung für den Flüssigkeits-führenden Hohlkolben (9) und den Flüssigkeitsauslass, in dem die Filter der Düse vorangestellt angeordnet sind. In der hier gezeigten Ausführungsform ist die Druckkammer im Wesentlichen kreiszylindrisch. Der Aufbau des Düsen- und Pumpenkammersystems ist in der favorisierten Ausführungsform derart gestaltet, dass das die Pumpenkammer bestimmende Zentralteil (23) auf der Seite der Hohlkolbens (9) eine zentrale Bohrung - stromabwärts, d.h. in Durchflussrichtung fortschreitend, vorzugsweise leicht konisch verengt - aufweist, die sowohl Filterbauteile aufnimmt, die Druckkammer (11) bildet und den Hohlkolben (9) und an einer entsprechend verbreiterten Stelle die zugehörige Dichtung (24) aufnimmt. Stromabwärts wird auf das Zentralteil (23) die Düsenbaugruppe (29) angebunden, welche das die Düse bildende mikrostrukturierte Bauteil (12) und diverse zugehörige Halterungs- oder Dichtungsbauteile enthält.
Vorzugsweise ist das in Flussrichtung als Erstes auf die Druckkammer (11) folgende Filterbauteil ein Grobfilter oder Vorfilter (27), dem sich direkt ein Feinfilter (28) anschließt. Weiter stromabwärts schließt sich das die Düse bildende mikrostrukturierte Bauteil (12) an, das nicht nur die eigentlichen Düsenkanäle (12d) sondern auch einen integrierten Feinstfilter (12f) enthält. Auf diese Weise fließt die Flüssigkeit im Gerät durch drei filternde Bauteile: einen Grobfilter oder Vorfilter (27), einen Feinfilter (28) und schließlich einen Feinstfilter (12f). Je nach Partikelaufkommen oder geforderter Partikelfreiheit der ausgebrachten Flüssigkeit ist auch der Einbau weiterer Filterelemente möglich. In einer favorisierten Ausführungsform setzt sich die Düse bzw. das mikrostrukturierte Bauteil (12), das sowohl die Düsenöffnungen (12e) als auch einen Feinstfilter (12f) beinhaltet, aus einer mikrostrukturierten Platte (12a), vorzugsweise aus Silizium, und einer die Strukturen abdeckenden Platte (12b), vorzugsweise aus Glas, zusammen. Die auf diese Weise eingeschlossene, über Mikrostrukturtechniken erzeugte Struktur bildet entlang der Strömungsrichtung hinter einem Einströmbereich (12c) zuerst einen als Strömungsfilter ausgelegten Feinstfilter (12f) und anschließend die Düsenkanäle (12d). Die Filterwirkung wird durch eine spezielle Anordnung von festen Stegen und Durchlässen erzielt. Besonders bevorzugt ist die zickzackförmige Anordnung von Stegreihen mit feinsten Durchlässen mit fertigungsbedingt rechteckigem Profil. Die Durchlassbreiten betragen hierbei nur wenige Mikrometer - vorzugsweise werden Teilchen bis zu einer Größe von etwa 2 Mikrometern aus der Flüssigkeit entfernt, bevor diese in die Düsenkanäle eintritt und später nach Zerstäubung von einem Benutzer des Inhalators eingeatmet wird. Weitere Details möglicher Strukturen für das in der Düsenbaugruppe (29) eingebaute mikrostrukturierte Bauteil (12) bzw. Feinstfilter (12f) werden in den Schriften WO94/07607A1, WO99/16530A1, WO05/000476A1, WO07/101557A2 und WO08/138936A2 offenbart.
Das Gesamtsystem aus Druckerzeuger (5) mit Antriebsfeder (7), Vorfilter (27), Feinfilter (28) und mikrostrukturiertem Bauteil (12) ist vorzugsweise so aufgebaut, dass bei der Sprühnebelerzeugung nicht nur an die Lungengängigkeit angepasste Tröpfchengrößen gebildet werden, sondern dass die Sprühnebelwolke selbst so lange anhält, dass der Patient seine Einatmung leicht an sie anpassen kann. Bevorzugt werden hierbei Sprühzeiten von 0,5 bis 2 Sekunden, insbesondere von 1 bis 2 Sekunden. Die Wahl des Filtersystems und der Filterschwellen im Zerstäuber beeinflusst die Länge der Sprühzeit. Insbesondere hat es sich hinsichtlich der insgesamt resultierenden Sprühzeit bei dem vorliegenden Filtersystem aus Vorfilter (27), Feinfilter (28) und Feinstfilter (12f) als vorteilhaft erwiesen, einen Feinfilter (28) einzusetzen der im vorliegenden Hochdrucksystem einen Druckabfall von 30-70 bar verursacht und so zu einer Verlängerung der Sprühzeit beiträgt. Der Druckabfall hängt hierbei mit der Porengröße des Filters zusammen: Je kleiner die Filterschwelle bei gleich bleibender Geometrie, d.h. je kleiner die Porengröße, umso höher ist der Druckabfall am Filter.
Die Filterbauteile des Darstellungsbeispiels unterscheiden sich nicht nur hinsichtlich ihrer Filterschwelle, sondern sind auch hinsichtlich ihrer Beschaffenheit, Struktur und Materialien verschiedenartig. Die drei filternden Bauteile des Geräts sind ein Kunststofffilter, ein Metallfilter und ein mikrostrukturiertes Bauteil, das wie oben beschrieben vorzugsweise aus einem Glas-Silizium-Verbund ist. Für den Vorfilter (27) und für den Feinfilter (28) werden also vorzugsweise andere Materialien als für den Feinstfilter (12f) ausgewählt. Besonders bevorzugt wird ein Vorfilter (27) aus Kunststoff wie beispielsweise aus einem zu den meisten medizinischen Formulierungen chemisch verträglichen Polyolefin-Material wie Polyethylen (PE), Polypropylen (PP) oder Polytetrafluorethylen (PTFE). Weiterhin ist der Einsatz eines Vorfilters (27) aus einem modifizierten Polyolefin, wie z.B. Metallocen-PP möglich, wobei man die speziellen Adsorptionseigenschaften des Materials nutzen kann. Vorzugsweise besteht der Vorfilter (27) hierbei aus verpresstem Kunststoffgranulat oder Sintermaterial, in diesem Fall besonders bevorzugt aus Polyethylen-Sintermaterial. Für den Feinfilter (28) wird der Einsatz eines metallischen Filters bevorzugt. In der hier dargestellten Ausführungsform besteht der Feinfilter (28) vorzugsweise aus Sintermetall insbesondere in Form von feinporig verpressten Metallpartikeln aus Edelstahl oder besonders bevorzugt aus Titan. Im Strömungsverlauf zwischen einem Kunststofffilter mit beispielsweise 9-15 Mikrometer durchmessenden Porengrößen und einem Feinstfilter (12f) von Durchlassbreiten von 2 Mikrometern wird ein Feinfilter mit vergleichsweise mittlerer Porengröße im Bereich von 5 bis 3 Mikrometer bevorzugt. Dementsprechend wird die Porengröße des Filters aus Metall derart klein gewählt, dass die Porendurchmesser kleiner als 5 Mikrometer sind. Das hier dargestellte mehrstufige Filtersystem ist mit 2 Mikrometer Durchlassbreite am Feinstfilter (12f) so ausgelegt, dass auch sehr große Wirkstoffmoleküle oder Suspensionsbestandteile dem Strömungsweg folgen und durch Zerstäubung vom Benutzer inhaliert werden können. Alternativ ist es ebenfalls möglich, Feinfilter (28) oder Feinstfilter (12f) als so genannten Sterilfilter mit Porengrößen bis runter zu 0,05 Mikrometern oder wie in der Schrift WO08/138936A2 für Sterilanwendungen bevorzugt im Porengrößenbereich 0,2 bis 0,3 Mikrometer zu verwenden. Strömungsweg und Druckerzeuger müssen dann jeweils an die größeren Druckverluste angepasst werden, die an Sterilfiltern entstehen.
Es hat sich gezeigt, dass Filter mit geeigneten Porengrößen kleiner als 5 Mikrometer in Sinterverfahren aus solchen Metallpulvern hergestellt werden können, die aus oberflächenzerklüfteten, so genannten spratzigen Teilchen bestehen. Durch die Verwendung eines solchen spratzig fraktionierten Metallpulvers können besonders kleinen Porengrößen erzielt werden, so dass die Herstellung eines Metallfilters mit dem besonders bevorzugten Porendurchmesser von 4 Mikrometer möglich ist. Besonders bevorzugt ist die Verwendung von Titan als spratzig fraktioniertes Metallpulver, d.h. die Verwendung von Titan-Sintermetall als Filtermaterial. Die Verwendung von unedleren Metallen kann insbesondere aufgrund der großen Oberfläche eines Filters zur schnellen Bildung von Korrosionen führen, insbesondere wenn sich im gleichen fluidischen System Bauteile aus hochwertigen Edelmetallen befinden, wie z.B. der bevorzugte Hohlkolben (9) im Zerstäuber des Darstellungsbeispiels. Die Verwendung eines Edelmetalls wie Titan, Gold oder Platin als Feinfilter (28) in einem mit Flüssigkeit betriebenen medizinischen System - die Verwendung von Titan stellt hierbei die kostengünstigste Möglichkeit dar - hat des weiteren den Vorteil, dass es inert und somit chemisch verträglich zu den meisten medizinischen Wirkstoff- und Formulierungs-Substanzen ist. Auf diese Weise verändert der Filter weder die Formulierung noch kann er von dieser verändert, insbesondere korrodiert werden. Letzteres ist insbesondere wichtig bei der Verwendung von sauren medizinischen Formulierungen wie bevorzugt im pH-Bereich von 3,5 bis 9 und insbesondere von pH 3,5 bis 6, die ihrerseits zur Korrosion von unedlen Filtermetallen führen können. Das hier vorgestellte Filtersystem ist auf die Filterung von solch sauren Formulierungen angepasst.
Die spezielle Auswahl der Filtermaterialien und der Filtercharakteristiken ist letztendlich abhängig vom speziellen Anwendungszweck oder von der Wahl der einzusetzenden Flüssigkeit (2) bzw. medizinischen Formulierung und ihrer Verträglichkeiten mit anderen Materialien. Die hier genannten Materialien und Einbaukonzepte decken bereits ein breites Anforderungsfeld ab. Insbesondere eignen sich Geräte mit einem Filtersystem dieser Art zur Verabreichung einer flüssigen medizinischen Formulierung, die eine Substanz mit geringem Dampfdruck oder eine alkoholische Verbindung z.B. als Lösungsmittel enthält. Im Darstellungsbeispiel weist das Gerät ein Zentralteil (23) auf, in dem beide Filter bzw. der Vorfilter (27) und der Feinfilter (28) nach einer Druckkammer (11) oder Dosierkammer angeordnet sind, die von selbigem Zentralteil (23) gebildet wird. Die Filter sind also zwischen Druckkammer (11) und Düsenkanälen (12d) angeordnet. Sowohl Feinfilter (28) als auch Vorfilter (27) bzw. Grobfilter haben eine kreiszylindrische oder zumindest teilweise konusförmige Gestalt. Besonders bevorzugt hat mindestens ein Filter eine leicht von der kreiszylindrischen Form abweichende Form mit konisch zulaufenden Seitenwänden. Fig. 5 zeigt eine besonders bevorzugte Geometrie des Feinfilters (28). Hiernach weist der Feinfilter (28) an der Eintrittsseite der Flüssigkeit eine kreizylindrische Struktur auf, die sich über ein Drittel bis Hälfte der Gesamtlänge des Feinfilters (28) in Form eines konstanten kreisförmigen Durchmessers erstreckt. An diesen kreiszylindrischen Bereich des Feinfilters (28) schließt sich ein konischer Bereich an, so dass der kreisförmige Durchmesser des Feinfilters (28) mit dem Strömungsverlauf kleiner wird. Am Strömungsauslass des Feinfilters (28) weist dieser erneut einen schmalen kreiszylindrischen Bereich auf. Durch diese Kombination aus zylindrischen und konisch zulaufenden Bereichen der Seitenwände werden gleichzeitig eine gute Montierbarkeit und eine gute Abdichtung des Feinfilters an der Innenwand des Zentralteils (23) auch bei der Verwendung von metallischen Materialien erzielt.
Gemäß einer weiteren, hier nicht anhand von Figuren dargestellten Ausführungsform können Vorfilter (27) und/oder Feinfilter (28) auch eine becherartige Form aufweisen. Ein solcher becherartiger Filter wird so in das Gerät eingebaut, dass die geöffnete Seite der Becherstruktur in Richtung Druckkammer zeigt. Die stromaufwärts zeigende Becherstruktur von Vorfilter (27) und/oder Feinfilter (28) weist eine gegenüber dem kreiszylindrischen Filtertyp deutlich vergrößerte Eintrittsoberfläche auf, an der mehr Partikel bereits bei Erreichen des Filters abgeschieden werden können. Dies ist insbesondere für Systeme von Bedeutung, bei denen es zur Bildung vieler insbesondere großer Partikel kommt, da für diese so die Filterkapazität erhöht werden kann.
Gemäß wiederum einer weiteren, hier nicht dargestellten Ausführungsform kann der Vorfilter (27) mit dem Feinfilter (28) in Form eines Bauteils mit gestufter Filterwirkung kombiniert werden. Im Falle der hier bevorzugten Materialien werden hierbei die Filtersegmente zusammengesintert.
Im Darstellungsbeispiel weisen beide Filter Abmessungen kleiner fünf und insbesondere zwischen 1 und 2 Millimetern auf, so dass sie sich gut in miniaturisierte Fluidsysteme wie den miniaturisierten Hochdruckzerstäuber einbauen lassen. Die Filter werden bei der Montage des Zerstäubers (1) hintereinander in die zugehörige zentrale Bohrung im Zentralteil (23) eingeschoben und bilden am Ende der Druckkammer (11) eine Presspassung mit der Innenwand des Zentralteils (23). Vorfilter (27) und Feinfilter (28) werden somit ohne weitere Bauteile zur Fixierung im Zentralteil (23) gehalten. Zusätzlich oder alternativ zur Presspassung und/oder kraftschlüssigen Verbindung kann das Zentralteil (23) die Filter auch durch eine innere Querschnittsverengung halten, so dass die Filter in Strömungsrichtung insbesondere in Strömungsrichtung fixiert werden. Die Materialauswahl der Filter ist auch von Bedeutung für ihre Einbausituation: Besonders bevorzugt folgt ein aus Metall gefertigter Filter, hier der Feinfilter (28) im Strömungsverlauf auf den aus vergleichsweise weichen Kunststoff gefertigten Filter, hier den Vorfilter (27). Dies hat insbesondere für die Anwendung in einem Hochdrucksystem den Vorteil, dass der Filter aus Metall eine Rückhaltefunktion für den Filter aus Kunststoff übernehmen kann. Je nach geometrischer Auslegung der auch fertigungsbedingten Durchführungen im Zentralteil könnte ein weicher Kunststofffilter unter hohem Flüssigkeitsdruck verformt, gegebenenfalls beschädigt oder aus ihrer dicht sitzenden Position herausgedrängt werden. Dies wird durch den Gegenhalt durch den vergleichsweise formstabilen metallischen Filter verhindert.
Die Wahl eines harten Metalls wie Titan als Material für den Feinfilter (28) hat beim Einbau in ein aus Kunststoff gefertigtes Zentralteil (23) den Vorteil, dass sich der feste, metallische Feinfilter (28) bei der konischen Verpressung in ein oberflächenweichere Kunststoffmaterial des Zentralteil (23) derart hineindrückt, dass es an seinen Seiten nicht zur Bildung von Bypässen kommen kann. Im bevorzugten Montageverfahren, bei dem der Feinfilter (28) vor dem Vorfilter (27) in die gleiche Durchführung am Zentralteil (23) eingesetzt wird, ist es vorteilhaft, dass das Zentralteil (23) oberflächenweicher als der Feinfilter (28), aber oberflächenhärter als der Vorfilter (27) ist. Ist der Vorfilter (27) aus einem besonders oberflächenweichen Material wie beispielsweise PE oder PP so bildet sich beim Verpressen im härteren Zentralteil ebenfalls ein besonders dichter Sitz gegenüber der Seitenwand, so dass auch hier die Bildung von Bypässen verhindert wird. Besonders bevorzugt besteht das Zentralteil (23) aus einem festen, druckstabilen Kunststoff wie insbesondere PEEK.
Betrachtet man das Montageverfahren zum Gerät insgesamt, so werden die Filter durch die spätere Druckkammer (11) hindurch in das Zentralteil (23) eingeführt und die Einführöffnung wird später durch den Kolben des Druckerzeugers (5) oder durch ein insbesondere als Hohlkolben (9) gestaltete Verbindungselement zur späteren Entnahme von Flüssigkeit (2) aus einem Behälter (3) verschlossen.
In einer - hier nicht im Bild dargestellten - weiteren Ausführungsform kann der Feinfilter (28) ein Metallgewebe-Filter sein, dessen Feinporigkeit durch das Übereinanderlegen von mehreren Lagen feinmaschiger Netze entsteht. Diese Netze können beispielsweise direkt im Spritzgussverfahren im Zentralteil (23) angespritzt werden. Alternativ wird das Gewebe als separates einbaufertiges Filterelement in einen Kunststoff wie TPE oder besonders bevorzugt ein Elastomer eingebettet, so dass ein einziges Bauteil nicht nur den Filter sondern auch sein zugehöriges Halteelement oder bevorzugt Dichtungselement beinhaltet.

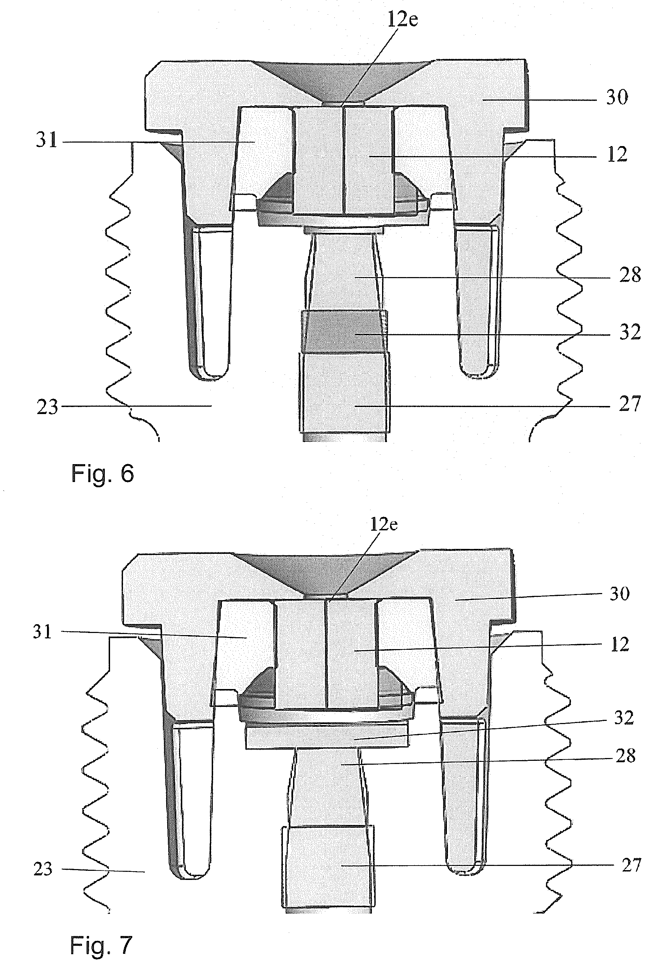

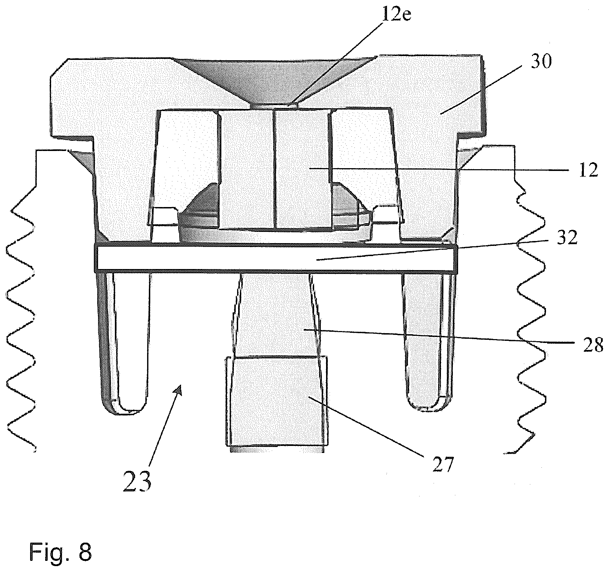

Figuren 6 bis 8 zeigen in vergrößerter Darstellung die Düsenbaugruppe (29) mit vorgeschaltetem Filtersystem in verschiedene Ausführungsformen, die im Vergleich zu dem in Figur 3 dargestellten Ausführungsbeispiel jeweils alle einen separaten Adsorptionsfilter (32) als zusätzlichen Filter zu Vorfilter (27), Feinfilter (28) und Feinstfilter (12f) enthalten. Neben den durch den Adsorptionsfilter (32) neu hinzu kommenden Aspekten, treffen die anhand von Figur 3 beschriebenen Aspekte auch bei diesen Ausführungsbeispielen zu. Hierbei ist der Adsorptionsfilter (32) wahlweise zwischen Vorfilter (27) und Feinfilter (28) (Figur 6) oder zwischen Feinfilter (28) und mikrostrukturiertem Bauteil (12) mit Feinstfilter (12f) und Düse angeordnet (Figur 7 und 8).
In dem Ausführungsbeispiel gemäß Figur 6 schließt sich, in Strömungsrichtung gesehen, an den Vorfilter (27) der Adsorptionsfilter (32) an, der zur Abscheidung von NanoPartikeln in einem Größenbereich << 100 Nanometer dient, die sich an den Auslässen der Düse am mikrostrukturierten Bauteil (12) anlagern können. Der Adsorptionsfilter (32) ist aus einer mittels Aluminiumoxiden funktionalisierten Glasfaser als Trägerfaser gefertigt und kraftschlüssig zwischen dem Vorfilter (27) und einem Feinfilter (28) - vorzugsweise aus Titan - zur Filtrierung von Teilchen bis zu einer Größe von ca. 3 bis 5 µm gehalten. Das die Düse bildende, mikrostrukturierte Bauteil (12) ist im Darstellungsbeispiel ein Glas-Silizium-Verbund. Das mikrostrukturierte Bauteil (12) wird mittels eines Halters (30) mit einer zugehörigen Dichtung (31) am Zerstäuber (1) fixiert. Dieses mikrostrukturierte Bauteil (12) enthält nicht nur die Düse mit den Düsenöffnungen (12e) zur Erzeugung von mikroskopischen Flüssigkeitsstrahlen sondern auch einen Feinstfilter (12f), der entsprechend Figur 4 vorzugsweise als zick-zack-förmiger Strömungsfilter ausgebildet ist und zur Entfernung von Teilchen bis zu einer Größe von etwa 2 µm aus der Flüssige medizinische Formulierung im Strömungsverlauf vor den Düsenkanälen (12d) und/oder den Düsenöffnungen (12e) dient. Durch die Verbindung der Mikrostrukturen von Düse und Feinstfilter (12f) in einem mikrostrukturierten Bauteil (12) ist der Feinstfilter (12f) dem Feinfilter (28) in Strömungsrichtung nachgeordnet. Wie in Figur 6 gezeigt wird, kann der Adsorptionsfilter (32) zwischen zwei Filtersystemen formschlüssig und/oder kraftschlüssig eingeschlossen werden. Im dargestellten Beispiel wird er zwischen Vorfilter (27) und Feinfilter (28) im Zentralteil (23) eingepresst.

In alternativen Ausgestaltungen entsprechend der Figuren 7 und 8 ist dem Vorfilter (27) der Feinfilter (28) nachgeordnet und der Adsorptionsfilter (32) befindet sich unmittelbar zwischen dem Feinfilter (28) und dem Feinstfilter (12f). In den gezeigten, bevorzugten Ausführungsformen aus Figur 7 und 8 ist der Adsorptionsfilter (32) als eine Filterscheibe ausgebildet, die mit ihrem Rand kraftschlüssig gehalten ist. Zweckmäßigerweise ist der Rand der Filterscheibe entweder durch eine Dichtung (31) des den Feinstfilter (12f) und Düse umfassenden mikrostrukturierten Bauteils (12) oder durch einen Halter (30) in der Düsenbaugruppe (29) (Fig. 8) oder durch das mikrostrukturierte Bauteil (12) selbst (Fig. 7) gehalten. Aufgrund des gegenüber dem Feinfilter (28) vergrößerten Durchmessers der Filterscheibe und dem direkten Aufeinanderfolgen von Feinfilter (28) und Filterscheibe, ist der Rand des Adsorptionsfilters (32) außerhalb des Strömungsweges. Dadurch dass sich der Rand des Adsorptionsfilters (32) nicht im Strömungsweg befindet, besteht keine Gefahr, dass Bestandteile des Adsorptionsfilters (32) während des Gebrauchs durch Bypässe emittieren, was in der Regel an der randseitigen Schnittkante erfolgen kann. Der wesentliche Unterschied, der zwischen den beiden Einbausituationen gemäß Figur 7 und 8 besteht, ist der Durchmesser des Adsorptionsfilters (32), der bei Halterung durch das mikrostrukturierte Bauteil (12) kleiner ausfällt als bei Halterung durch das mikrostrukturierte Bauteil (12) umfassende Bauteile wie den Halter (30) und die Dichtung (31). Bei gängigen Abmessungen solch eines mikrostrukturierten Bauteils (12) resultieren für beide Szenarien für den Adsorptionsfilter Durchmesser von etwa 2 bis 5 mm, vorzugsweise ca. 3 mm bei Halterung durch das mikrostrukturierte Bauteil (12) und von etwa 3 bis 10 mm, bevorzugt ca. 6 mm bei Halterung durch Halter (30) und/oder Dichtung (31). Im Ausführungsbeispiel gemäß Figur 8 weist der zwischen dem Feinfilter (28) und dem Feinstfilter (12f) vorgesehene Adsorptionsfilter (32) jedenfalls einen derart großen Durchmesser auf, dass er randseitig durch den Halter (30) des mikrostrukturierten Bauteils (12) geklemmt ist.

Die Idee, verschiedenartig beschaffene und verschieden wirkende Filterelemente hintereinander zu schalten, ist auf viele Geräte, in denen Flüssigkeiten gefördert oder transportiert werden, übertragbar. Der vorschlagsgemäße Zerstäuber (1) arbeitet zwar insbesondere rein mechanisch. Das hier vorgestellte Filtersystem ist jedoch nicht auf die Anwendung in rein mechanischen Geräten zur Ausbringung einer Flüssigkeit begrenzt. Es kann beispielsweise ebenso in Systemen eingesetzt werden, in denen die Ausbringung der Flüssigkeit durch Treibgas oder durch elektrische, hydraulische oder andere Pumpen angetrieben ist. Begriffe wie "Druckerzeuger" sind also allgemein zu verstehen. In diesem Sinne kann die vorliegende Erfindung auch Gebiets-übergreifend verwendet werden; selbst Anwendungen über den medizinischen Bereich hinaus sind möglich.

Der hier dargestellte Zerstäuber dient zwar speziell der Ausgabe einer flüssigen medizinischen Formulierung als inhalierfähiges Aerosol und eignet sich für die Ausbringung sowohl wässriger als auch bevorzugt alkoholischer, insbesondere ethanolischer medizinischer Formulierungen.
Bevorzugte Inhaltsstoffe der vorzugsweise flüssigen medizinischen Formulierung sind insbesondere in den Schriften WO09/047173A2 und WO09/115200A1 aufgeführt, die hiermit diesbezüglich vollumfänglich als Referenz eingeführt werden. Insbesondere kann es sich bei dem in diesen Schriften beschriebenen Fluid um wässrige oder nicht wässrige Lösungen, Mischungen, Formulierungen mit und ohne Lösungsmittel, wie Ethanol oder dergleichen, handeln.

**Bezugszeichenliste**

| | | | |
|---|---|---|---|
| 1 | Zerstäuber | 14 | Aerosol |
| 2 | Flüssigkeit | 16 | Gehäuseoberteil |
| 3 | Behälter | 17 | Gehäuseinnenteil |
| 5 | Druckerzeuger | 18 | Gehäuseunterteil |
| 6 | Halterung (für Behälter) | 19 | Sicherheitsverschluss |
| 7 | Antriebsfeder | 23 | Zentralteil |
| 8 | Sperring | 24 | Dichtung |
| 9 | Hohlkolben | 25 | Stützring |
| 10 | Rückschlagventil | | |
| 11 | Druckkammer | 27 | Vorfilter |
| 12 | Mikrostrukturiertes Bauteil | 28 | Feinfilter |
| 12a | Platte | 29 | Düsenbaugruppe |
| 12b | Platte | 30 | Halter |
| 12c | Einströmbereich | 31 | Dichtung |
| 12d | Düsenkanäle | 32 | Adsorptionsfilter |
| 12e | Düsenöffnungen | 40 | Taste |
| 12f | Feinstfilter | 41 | Zählwerk |

## Patentansprüche

1. Gerät zum Verabreichen einer flüssigen medizinischen Formulierung, die sich in einem in das Gerät eingesetzten Behälter (3) befindet und durch mindestens eine Düsenöffnung (12e) aus dem Gerät ausgebracht wird, wobei die flüssige medizinische Formulierung vor Durchströmen der mindestens einen Düsenöffnung (12e) durch einen Feinstfilter (12f) strömt, der von einem mikrostrukturierten Bauteil (12) gebildet wird, und wobei im Innern des Geräts im Strömungsweg der Flüssigkeit ein Vorfilter (27) vor dem mikrostrukturierten Bauteil (12) angeordnet ist, wobei im Strömungsweg der Flüssigkeit ein Feinfilter (28) zusätzlich zwischen Vorfilter (27) und dem mikrostrukturierten Bauteil (12) angeordnet ist, wobei der Feinfilter (28) zum Vorfilter (27) verschiedenartig ist, so dass an Vorfilter (27) und Feinfilter (28) jeweils Partikel unterschiedlicher Größe oder Art vor Eintritt der Formulierung in das mikrostrukturierte Bauteil (12) abgeschieden werden, und wobei Vorfilter (27), Feinfilter (28) und Feinstfilter (12f) aus unterschiedlichen Materialien bestehen, **dadurch gekennzeichnet, dass** einer der Filter aus Titan in Form eines Sintermetalls besteht.

2. Gerät nach Anspruch 1 **dadurch gekennzeichnet, dass** Vorfilter (27), Feinfilter (28) und Feinstfilter (12f) unterschiedliche Porengrößen aufweisen und so angeordnet sind, dass die Porengröße im Strömungsverlauf kleiner wird.

3. Gerät nach einem der vorherigen Ansprüche **dadurch gekennzeichnet, dass** der Feinfilter (28)aus Sintermetall in Form von feinporig verpressten Metallpartikeln aus Titan besteht.

4. Gerät nach einem der vorherigen Ansprüche **dadurch gekennzeichnet, dass** die Porengrößen des Filters aus Titan derart klein sind, dass die Porendurchmesser kleiner als 5 Mikrometer sind.

5. Gerät nach einem der vorherigen Ansprüche **dadurch gekennzeichnet, dass** der Vorfilter (27) ein Kunststofffilter ist, und das mikrostrukturiertes Bauteil (12) vorzugsweise aus einem Glas-Silizium-Verbund ist.

6. Gerät nach einem der Ansprüche 1 oder 2 **dadurch gekennzeichnet, dass** der Feinfilter (28) ein Filter ist, dessen Wirkungsweise vorrangig durch Adsorptionseffekte und weniger durch Größenausschuss bestimmt ist, wobei sich an der Oberfläche des Filters Partikel, insbesondere Nanopartikel, aus einer den Filter durchströmenden Flüssigkeit aufgrund intermolekularer Wechselwirkungen anlagern können.

7. Gerät nach einem der Ansprüche 1 bis 5 **dadurch gekennzeichnet, dass** ein Adsorptionsfilter (32) im Strömungsweg zwischen Vorfilter (27) und Feinstfilter (12f) angeordnet ist, wobei die Wirkungsweise des Adsorptionsfilters **dadurch gekennzeichnet ist, dass** sich an der Oberfläche des Filters Partikel, insbesondere Nanopartikel, aus einer den Filter durchströmenden Flüssigkeit aufgrund intermolekularer Wechselwirkungen anlagern können.

8. Gerät nach Anspruch 7 **dadurch gekennzeichnet, dass** der Adsorptionsfilter (32) als eigenständiges Bauteil entweder zwischen dem Vorfilter (27) und dem Feinfilter (28) oder zwischen dem Feinfilter (28) und dem Feinstfilter (12f) angeordnet ist.

9. Gerät nach Anspruch 7 oder 8 **dadurch gekennzeichnet, dass** der Adsorptionsfilter (32) als eine Filterscheibe ausgebildet ist, die mit ihrem Rand kraftschlüssig gehalten ist.

10. Gerät nach Anspruch 9 **dadurch gekennzeichnet, dass** der Rand der Filterscheibe entweder durch ein Bauteil, welches das mikrostrukturierte Bauteil (12) oder die Düse in seiner Einbauposition abdichtet oder fixiert, oder durch das mikrostrukturierte Bauteil (12) oder die Düse selbst gehalten ist oder zwischen Vorfilter (27) und Feinfilter (28) eingeklemmt ist.

11. Gerät nach einem der Ansprüche 7 bis 10 **dadurch gekennzeichnet, dass** der Adsorptionsfilter (32) aus Glas-, Zellulose-, Kohle- oder Polymerfaser gefertigt ist.

12. Gerät nach einem der Ansprüche 7 bis 11 **dadurch gekennzeichnet, dass** der Adsorptionsfilter (32) eine mit Aluminiumoxid funktionalisierte Oberfläche aufweist.

13. Gerät nach einem der Ansprüche 7 bis 11 **dadurch gekennzeichnet, dass** der Adsorptionsfilter (32) eine mit quartären Ammoniumgruppen funktionalisierte Oberfläche aufweist.

14. Gerät nach einem der vorherigen Ansprüche **dadurch gekennzeichnet, dass** Vorfilter (27) und Feinfilter (28) kreiszylindrische oder zumindest teilweise konusförmige Gestalt haben.

15. Gerät nach einem der vorherigen Ansprüche **dadurch gekennzeichnet, dass** die zu verabreichende flüssige medizinische Formulierung eine Substanz mit geringem Dampfdruck oder eine alkoholische Verbindung enthält.

16. Gerät nach einem der vorherigen Ansprüche **dadurch gekennzeichnet, dass** die zu verabreichende flüssige medizinische Formulierung einen pH-Wert zwischen 3,5 und 9 aufweist.

17. Gerät nach einem der vorherigen Ansprüche **dadurch gekennzeichnet, dass** die flüssige medizinische Formulierung als Aerosol insbesondere als inhalierfähiges Aerosol ausgegeben wird.

18. Gerät nach einem der vorherigen Ansprüche **dadurch gekennzeichnet, dass** das Gerät eine Druckkammer (11) beinhaltet und die Filter zwischen der Druckkammer (11) und der mindestens einen Düsenöffnung (12e) und/oder Düsenkanälen (12b) angeordnet sind.

19. Gerät nach einem der vorherigen Ansprüche **dadurch gekennzeichnet, dass** das Gerät ein Zentralteil (23) aufweist, in dem Vorfilter (27) und Feinfilter (28) nach einer Druckkammer (11) oder einer Dosierkammer angeordnet sind, wobei Vorfilter (27) und Feinfilter (28) ohne weitere Bauteile zur Fixierung im Zentralteil (23), insbesondere durch eine vom Zentralteil (23) selbst gebildete innere Querschnittsverengung und/oder durch eine Presspassung, gehalten werden.

20. Verfahren zur Montage eines Geräts nach einem der Ansprüche 18 bis 19 **dadurch gekennzeichnet, dass** Feinfilter (28) und Vorfilter (27) durch die spätere Druckkammer (11) hindurch in ein Zentralteil (23) eingeführt werden und die Einführöffnung durch den Kolben eines Druckerzeugers (5) oder durch ein insbesondere als Hohlkolben (9) gestaltetes Verbindungselement zur späteren Entnahme von Flüssigkeit (2) aus einem Behälter (3) verschlossen wird.

## Claims

1. Device for administering a liquid medical formulation which is held in a container (3) inserted in the device and is expelled from the device through at least one nozzle opening (12e), wherein, before flowing through the at least one nozzle opening (12e), the liquid medical formulation flows through a very fine filter (12f) which is formed by a microstructured component (12), and wherein a pre-filter (27) is arranged in front of the microstructured component (12) inside the device, in the flow path of the liquid, wherein a fine filter (28) is additionally arranged between the pre-filter (27) and the microstructured component (12) in the flow path of the liquid, wherein the fine filter (28) is different from the pre-filter (27), so that particles of different sizes or natures are deposited on the pre-filter (27) and fine filter (28) in each case before the formulation enters the microstructured component (12), and wherein the pre-filter (27), fine filter (28) and very fine filter (12f) are made of different materials, **characterised in that** one of the filters is made of titanium in the form of a sintered metal.

2. Device according to claim 1, **characterised in that** pre-filter (27), fine filter (28) and very fine filter (12f) have different pore sizes and are arranged so that the pore size decreases in the direction of flow.

3. Device according to one of the preceding claims, **characterised in that** the fine filter (28) is made of sintered metal in the form of fine-pore pressed metal particles of titanium.

4. Device according to one of the preceding claims, **characterised in that** the pore sizes of the titanium filter are so small that the pore diameters are less than 5 microns.

5. Device according to one of the preceding claims, **characterised in that** the pre-filter (27) is a plastics filter, and the microstructured component (12) is preferably made of a glass/silicon composite.

6. Device according to one of claims 1 or 2, **characterised in that** the fine filter (28) is a filter the mode of action of which is determined predominantly by adsorption effects and less by size exclusion, while particles, particularly nanoparticles, from a liquid flowing through the filter are able to accumulate on the surface of the filter as a result of intermolecular interactions.

7. Device according to one of claims 1 to 5, **characterised in that** an adsorption filter (32) is arranged in the flow path between pre-filter (27) and very fine filter (12f), the mode of action of the adsorption filter being **characterised in that** particles, particularly nanoparticles, from a liquid flowing through the filter are able to accumulate on the surface of the filter as a result of intermolecular interactions.

8. Device according to claim 7, **characterised in that** the adsorption filter (32) is arranged as an independent component either between the pre-filter (27) and the fine filter (28) or between the fine filter (28) and the very fine filter (12f).

9. Device according to claim 7 or 8, **characterised in that** the adsorption filter (32) is embodied as a filter disc which is frictionally secured by its edge.

10. Device according to claim 9, **characterised in that** the edge of the filter disc is held either by a component that seals off or fixes the microstructured component (12) or the nozzle in its installed position, or by the microstructured component (12) or the nozzle itself, or is clamped in position between the pre-filter (27) and fine filter (28).

11. Device according to one of claims 7 to 10, **characterised in that** the adsorption filter (32) is made of glass, cellulose, carbon or polymer fibres.

12. Device according to one of claims 7 to 11, **characterised in that** the adsorption filter (32) comprises a surface functionalised with aluminium oxide.

13. Device according to one of claims 7 to 11, **characterised in that** the adsorption filter (32) has a surface functionalised with quaternary ammonium groups.

14. Device according to one of the preceding claims, **characterised in that** pre-filter (27) and fine filter (28) have a circular-cylindrical or at least partially conical configuration.

15. Device according to one of the preceding claims, **characterised in that** the liquid medical formulation that is to be administered contains a substance with a low vapour pressure or an alcoholic compound.

16. Device according to one of the preceding claims, **characterised in that** the liquid medical formulation that is to be administered has a pH of between 3.5 and 9.

17. Device according to one of the preceding claims, **characterised in that** the liquid medical formulation is delivered as an aerosol, particularly an inhalable aerosol.

18. Device according to one of the preceding claims, **characterised in that** the device contains a pressure chamber (11) and the filters are arranged between the pressure chamber (11) and the at least one nozzle opening (12e) and/or nozzle channels (12b).

19. Device according to one of the preceding claims, **characterised in that** the device comprises a central part (23) in which pre-filter (27) and fine filter (28) are arranged downstream of a pressure chamber (11) or a metering chamber, wherein pre-filter (27) and fine filter (28) are held in the central part (23) without any additional components for securing them, more particularly are held by a reduction in the internal cross-section formed by the central part itself (23), and/or by a press-fit.

20. Method of assembling a device according to one of claims 18 to 19, **characterised in that** fine filter (28) and pre-filter (27) are inserted into a central part (23) through what is to be the pressure chamber (11) and the insertion opening is closed off by the piston of a pressure generator (5) or by a connecting element formed particularly as a hollow piston (9) for subsequently withdrawing liquid (2) from a container (3).

## Revendications

1. Dispositif pour l'administration d'une formulation médicale liquide, qui se trouve dans un contenant (3) inséré dans le dispositif et qui est distribuée depuis le dispositif par au moins une ouverture de buse (12e), dans lequel la formulation médicale liquide traverse, avant de passer par l'au moins une ouverture de buse (12e), un filtre ultrafin (12f), qui est formé par un composant (12) microstructuré, et dans lequel un préfiltre (27) est disposé, à l'intérieur de l'appareil, dans le trajet d'écoulement du liquide, avant le composant (12) microstructuré,
dans lequel un filtre fin (28) est disposé, dans le trajet d'écoulement du liquide, en supplément entre le préfiltre (27) et le composant (12) microstructuré, dans lequel le filtre fin (28) est d'un type différent par rapport au préfiltre (27) de sorte que respectivement des particules de taille ou de type différents soient filtrées, avant l'entrée de la formulation dans le composant (12) microstructuré, au niveau du préfiltre (27) et du filtre fin (28), et dans lequel le préfiltre (27), le filtre fin (28) et le filtre ultrafin (12f) sont constitués de matériaux différents,
**caractérisé en ce que** l'un des filtres est constitué de titane sous la forme d'un métal fritté.

2. Dispositif selon la revendication 1, **caractérisé en ce que** le préfiltre (27), le filtre fin (28) et le filtre ultrafin (12f) présentent des tailles de pores différentes et sont disposés de telle sorte que la taille des portes devient plus petite au fur et à mesure de l'écoulement.

3. Dispositif selon l'une quelconque des revendications précédentes, **caractérisé en ce que** le filtre fin (28) est constitué de métal fritté sous la forme de particules métalliques compressées à pores fins, composées de titane.

4. Dispositif selon l'une quelconque des revendications précédentes, **caractérisé en ce que** les tailles des portes du filtre composé de titane sont petites de telle manière que les diamètres de pores sont inférieurs à 5 micromètres.

5. Dispositif selon l'une quelconque des revendications précédentes, **caractérisé en ce que** le préfiltre (27) est un filtre en plastique, et le composant (12) microstructuré est fait de préférence d'un composé de verre et de silicium.

6. Dispositif selon l'une quelconque des revendications 1 ou 2, **caractérisé en ce que** le filtre fin (28) est un filtre dont l'action est définie dans une large mesure par des effets d'adsorption et moins par l'exclusion de tailles, dans lequel des particules, en particulier des nanoparticules, issues d'un liquide traversant le filtre peuvent s'accumuler au niveau de la surface du filtre du fait d'interactions intermoléculaires.

7. Dispositif selon l'une quelconque des revendications 1 à 5, **caractérisé en ce qu'**un filtre d'adsorption (32) est disposé dans le trajet d'écoulement entre le préfiltre (27) et le filtre ultrafin (12f), dans lequel l'action du filtre d'adsorption est **caractérisée en ce que** des particules, en particulier des nanoparticules, issues d'un liquide traversant le filtre peuvent s'accumuler au niveau de la surface du filtre du fait d'interactions intermoléculaires.

8. Dispositif selon la revendication 7, **caractérisé en ce que** le filtre d'adsorption (32) en tant que composant autonome est disposé soit entre le préfiltre (27) et le filtre fin (28) soit entre le filtre fin (28) et le filtre ultrafin (12f).

9. Dispositif selon la revendication 7 ou 8, **caractérisé en ce que** le filtre d'adsorption (32) est réalisé sous la forme d'un disque filtrant, qui est maintenu à force par son bord.

10. Dispositif selon la revendication 9, **caractérisé en ce que** le bord du disque filtrant est maintenu soit par un composant, qui étanchéifie ou fixe le composant (12) microstructuré ou la buse dans sa position de montage, soit par le composant (12) microstructuré ou par la buse elle-même ou est introduit et serré entre le préfiltre (27) et le filtre fin (28).

11. Dispositif selon l'une quelconque des revendications 7 à 10, **caractérisé en ce que** le filtre d'adsorption (32) est fabriqué à partir d'une fibre de verre, de cellulose, de carbone ou de polymère.

12. Dispositif selon l'une quelconque des revendications 7 à 11, **caractérisé en ce que** le filtre d'adsorption (32) présente une surface rendue fonctionnelle avec de l'oxyde d'aluminium.

13. Dispositif selon l'une quelconque des revendications 7 à 11, **caractérisé en ce que** le filtre d'adsorption (32) présente une surface rendue fonctionnelle avec des groupes d'ammonium quaternaires.

14. Dispositif selon l'une quelconque des revendications précédentes, **caractérisé en ce que** le préfiltre (27) et le filtre fin (28) ont une configuration cylindrique circulaire ou au moins en partie une configuration en forme de cône.

15. Dispositif selon l'une quelconque des revendications précédentes, **caractérisé en ce que** la formulation médicale liquide à administrer contient une substance avec une faible pression de valeur ou un composé alcoolisé.

16. Dispositif selon l'une quelconque des revendications précédentes, **caractérisé en ce que** la formulation médicale liquide à administrer présente une valeur pH comprise entre 3,5 et 9.

17. Dispositif selon l'une quelconque des revendications précédentes, **caractérisé en ce que** la formulation médicale est fournie sous la forme d'un aérosol, en particulier sous la forme d'un aérosol à inhaler.

18. Dispositif selon l'une quelconque des revendications précédentes, **caractérisé en ce que** le dispositif comporte une chambre de pression (11), et les filtres sont disposés entre la chambre de pression (11) et l'au moins une ouverture de buse (12e) et/ou des canaux de buse (12b).

19. Dispositif selon l'une quelconque des revendications précédentes, **caractérisé en ce que** l'Dispositif présente une partie centrale (23), dans laquelle le préfiltre (27) et le filtre fin (28) sont disposés après une chambre de pression (11) ou une chambre de dosage, dans lequel le préfiltre (27) et le filtre fin (28) sont maintenus, sans composants supplémentaires, aux fins de la fixation dans la partie centrale (23), en particulier par un rétrécissement de section transversale intérieur formé par la partie centrale (23) elle-même et/ou par un ajustement par pression.

20. Procédé servant à monter un dispositif selon l'une quelconque des revendications 18 à 19, **caractérisé en ce que** le filtre fin (28) et le préfiltre (27) sont introduits dans une partie centrale (23) en passant par la chambre de pression (11) ultérieure, et l'ouverture d'introduction est fermée par le piston d'un générateur de pression (5) ou par un élément de liaison configuré en particulier sous la forme d'un piston creux (9) afin de prélever ultérieurement du liquide (2) hors d'un contenant (3).
